(19) Europäisches Patentamt
European Patent Office
Office européen des brevets

(11) EP 2 648 662 B1

(12) **EUROPEAN PATENT SPECIFICATION**

(45) Date of publication and mention of the grant of the patent:
**15.04.2015 Bulletin 2015/16**

(21) Application number: **11799350.1**

(22) Date of filing: **12.12.2011**

(51) Int Cl.:
***A61F 5/44*** *(2006.01)*

(86) International application number:
**PCT/DK2011/050476**

(87) International publication number:
**WO 2012/076022 (14.06.2012 Gazette 2012/24)**

(54) **METHOD OF FORMING AN OPENING IN A BASE PLATE OF STOMA DEVICE**

VERFAHREN ZUR BILDUNG EINER ÖFFNUNG IN EINER BASISPLATTE EINER STOMAVORRICHTUNG

PROCÉDÉ DE FORMATION D'UNE OUVERTURE DANS LA PLAQUE-SUPPORT D'UN DISPOSITIF DE STOMIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.12.2010 JP 2010276450**

(43) Date of publication of application:
**16.10.2013 Bulletin 2013/42**

(73) Proprietor: **Coloplast A/S**
**3050 Humlebæk (DK)**

(72) Inventor: **KIYOSHI, Hori**
**Hyogo 651-2273 (JP)**

(56) References cited:
**EP-A1- 0 800 804**
**WO-A1-2006/122565**
**DE-A1- 4 015 186**
**DE-U1- 9 105 753**
**US-B1- 6 659 989**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 648 662 B1

## Description

### Technical Field

The present invention relates to a method of forming an opening in a base plate of a stoma device.

### Background Art

In colectomies, ileectomies, urethectomies, and similar surgical procedures, the skin of the abdomen etc. is formed with an opening for the discharge of faeces or urine. That is, a so-called "stoma". The patient formed with a stoma cannot control the discharge of faeces, so it becomes necessary to utilize a stoma device for collecting the faeces discharged from the stoma.

In general, a stoma device is provided with a base plate which is to be stuck to the skin around the stoma of the patient and a stoma pouch which is connected to this base plate and collects faeces discharged from the stoma. Stoma devices may be roughly classified into one-piece types and two-piece types. In a one-piece type of stoma device, the base plate and the stoma pouch are integrally formed and are unable to be detached from each other. On the other hand, in a two-piece type of stoma device, the base plate and the stoma pouch can be separately formed and be able to be detached from each other. For this reason, in this two-piece type of stoma device, it is possible to leave the base plate stuck to the skin and just replace the stoma pouch.

Here, in general, the shape etc. of a stoma differs for each patient. Therefore, when using such a stoma device, usually, a patient has to cut the base plate of the stoma device so that an opening of a shape matching his or her own stoma is formed in the base plate, then, after the base plate is cut in this way, has to stick the base plate to the skin around the stoma.

However, for example, if the patient is clumsy or has poor eyesight and the stoma is complicated in shape, it is often difficult for the patient to suitably cut the base plate. As a result, the shape of the opening formed in the base plate cut by the patient will become a shape different from the actual stoma.

Therefore, for example, in the method of production of a stoma device described in JP 10-28698, the shapes of the stoma of the patient and the skin around the stoma are electronically measured and the base plate is formed so as to have physical properties selected based on the measured shapes.

Moreover, WO 2006/122565 discloses a device for recording and transferring the contours of a wound or opening in tissue of a human being comprising a transparent polymer sheet having a first and a second surface, wherein the first surface faces the wound/opening and the second surface faces comprises a central portion and an edge porting wherein the edge porting is provided with an adhesive layer. The device is placed over the wound/opening and the contours are traced on the central portion of the device. The device is reversed and transferred to an appliance and an aperture is cut by the patient from the traced contours.

Further background art may be found in EP 0 800 804 which describes a method of production of a stoma device matching the shape of the stoma.

In order to relieve the patient of the burden of cutting the base plate, a method of using cardboard may be considered. In this method, first, the patient draws the shape of the stoma on a piece of cardboard and faxes or mails the thus drawn upon piece of cardboard to a cutting service. The cutting service receiving the fax or mail cuts the base plate so as to form in the base plate the same shape of opening as the shape drawn on the cardboard. Due to this, the patient can obtain a base plate formed with an opening of a shape similar to the stoma by just mailing or faxing a piece of cardboard on which the shape of his or her own stoma is drawn.

However, if the patient faxes a piece of cardboard on which the patient has drawn the shape of the stoma, sometimes the shape of the drawn stoma hole will differ at the sending side and receiving side of the fax. In this case, if cutting the base plate based on the shape of the stoma hole on the piece of cardboard sent by the fax, the base plate will be formed with an opening of a shape different from the shape intended by the patient. Further, when the patient has drawn the shape of his or her own stoma on the piece of cardboard by a relatively thick line, the opening will differ in size between the case of cutting the base plate along the inside of the line and the case of cutting the base plate along the outside of the line. Therefore, in this case as well, the base plate will be formed with an opening of a shape different from the shape intended by the patient.

If the shape of the opening formed in the base plate in this way differs from the shape intended by the patient, that is, if the shape of the opening formed in the base plate differs from the shape of the actual stoma, the opening of the base plate will become too small and part of the base plate will contact the stoma, or the opening of the base plate will become too large and the skin around the stoma will contact the faeces discharged from the stoma. This will cause injury to the stoma, inflammation of the skin, etc.

It is an object of one or more embodiments of the present invention to provide an alternative to the abovementioned methods.

Moreover, it is an object of one or more embodiments of the present invention, to provide a method for forming an opening of a shape which is close to or even precisely matching the stoma of a patient in a base plate of a stoma device.

**Description of Invention**

The present invention relates to a method of forming an opening (for receiving a stoma) in a base plate of a stoma device, the method of forming an opening comprising steps of: printing on a label a filled-in-part of the same shape as a hole of a shape formed on a template sheet and matching the stoma of a patient; sticking the label on which the filled-in-part has been printed on to a base plate; and a step of cutting the base plate along the contour of the filled-in-part of the label stuck to the base plate.

Preferred embodiments of the invention are defined in the dependent claims.

**Brief Description of the figures**

The invention will now be described with reference to the figures in which:

Fig. 1 is a view which illustrates a one-piece type of stoma device,

Fig. 2 is a partial cross-sectional view of a base plate,

Fig. 3 is a view which illustrates a two-piece type of stoma device,

Fig. 4 is a flowchart schematically showing the process for forming an opening in the present invention,

Fig. 5 is a plan view and a bottom view of a template sheet to be used in the method of forming an opening of the present invention,

Fig. 6 is a view which illustrates a cut template sheet,

Fig. 7 is a view which illustrates the cut-out of image data,

Fig. 8 is a view which illustrates filling in of image data,

Fig. 9 is a view which illustrates addition of cross lines,

Fig. 10 is a view which illustrates one example of a label to be stuck to a base plate,

Fig. 11 is a plan view which schematically illustrates a base plate, and

Fig. 12 is a plan view which schematically illustrates another base plate.

**Detailed description of the figures**

Below, referring to the drawings, embodiments of the present invention will be explained in detail. Note that, in the following explanation, similar component are assigned the same reference numerals.

Fig. 1 is a view which schematically illustrates a stoma device 11. The stoma device 11 is provided with a base plate (wafer) 12 to be stuck to the skin of a patient around a stoma, and a stoma pouch 13 which is connected to this base plate 12 and collects faeces discharged from the stoma. The base plate 12, as shown in Fig. 2, is provided with a plastic sheet 12a and an adhesive layer 12b which covers the entire front surface of this plastic sheet 12a. Furthermore, a protective sheet 12c is placed over the adhesive layer 12b. Further, the base plate 12 is provided with an opening 14 which is visible in Fig. 1. The stoma pouch 13 has an opening 15 at part of it. The edges of this opening 15 are connected to the back surface of the base plate 12 around the opening 14 of the base plate 12 so as to be unable to be detached.

In the thus configured stoma device 11, at the time of use, the protective sheet 12c of the base plate 12 is peeled off, then the adhesive layer 12b of the base plate 12 is used to stick the base plate 12 to the skin of the patient around the stoma. At this time, the base plate 12 is stuck to the skin so that the stoma of the patient is positioned inside of the opening 14 provided in the base plate 12. If the base plate 12 of the stoma device 11 is stuck to the skin of the patient in this way, the faeces discharged from the stoma of the patient will be collected in the stoma pouch 3 through the opening 14 of the base plate 12 and the opening 15 of the stoma pouch 3.

Note that, the above-mentioned stoma device 11 is a one-piece type in which the base plate and the stoma pouch are integrally formed, but as the stoma device, a two-piece type such as shown in Fig. 3 may also be used.

As shown in Fig. 3, a two-piece type of stoma device 21 is provided with a separately formed base plate 22 and stoma pouch 23. The base plate 22 is provided with an opening 24, while the back surface of the base plate 22 has a connecting ring 26 attached to it. On the other hand, around the opening 25 of the stoma pouch 23 as well, a connecting ring 27 is attached. These connecting rings 26 and 27 are attached to each other in a detachable manner. When the two are attached to each other, the faeces discharged from the stoma of the patient will be collected inside of the stoma pouch 23 through the opening 24 of the base plate 22 and the opening 25 of the stoma pouch 23 without leaking out from the connecting rings 26 and 27.

It will be appreciated that in the case of a two piece type the base plate and the stoma pouch may coupled to each other by other means than connecting rings. One example is by means of an adhesive.

In this regard, the shape etc. of a stoma differs with each patient, so it is necessary to make the opening 14,24 of the base plate 12,22 to be stuck on the skin around the stoma a suitable shape matching the shape of the stoma of the individual patient. Below, taking as an example the one-piece type stoma device 11, the method of forming an opening in the base plate 12 of the stoma device 11 will be explained.

Fig. 4 is a flowchart schematically showing the process for forming an opening in the present invention. As shown in Fig. 4, first, at step S11, the patient or caregiver (nurse, home helper, family member, etc.) cuts the template sheet 30 so as to form a hole of a shape matching the stoma of the patient in the template sheet 30. Note that, "a shape matching the stoma of the patient" means a shape identical to or substantially identical to the stoma of the patient or a shape of a width 1 to 2 mm larger than the stoma of the patient.

Fig. 5 is a view showing a template sheet 30 used in the method of the present invention. The template sheet 30 is formed by a transparent sheet material able to be cut by ordinary scissors, for example, plastic. In the example shown in Fig. 5, thick contour lines 30a, 30b, and 30c are printed on it. These contour lines 30a, 30b, and 30c form the same shapes as the contours of opening-forming regions of the base plate 12 in which openings are to be formed using this template sheet 30 (regions in the base plate 12 where openings can be formed and smaller than the opening 15 of the stoma pouch 13). In particular, in the example shown in Fig. 5, the contour line 30a is circular, while the contour lines 30b and 30c are elliptical. In this way, the template sheet 30 is printed with contour lines of the same shapes as opening-forming regions of the base plate 12, so the patient or caregiver can cut the template sheet 30 so that the opening cut in the base plate 12 does not stick out from the opening-forming region of the base plate 12 when finally cutting the base plate 12 based on the template sheet 30. In particular, in the present embodiment, since contour lines 30a, 30b, and 30c of the same shapes as the contours of the opening-forming regions of different base plates are printed, it is possible to use a single template sheet 30 for a plurality of different base plates.

Further, the template sheet 30 is printed with a plurality of graduation lines 30d provided at regular intervals at the inside of the contour line 30a and shaped concentrically with the contour line 30a. Lines similar to the graduation lines 30d are also printed on the base plate 12 as shown in Fig. 11 and are used for aligning the centres of the base plate 12 and the template sheet 30. Further, the template sheet 30 and the base plate 12 may be printed with positioning lines in addition to such graduation lines. These positioning lines are also used for aligning not only the centres of the template sheet 30 and base plate 12 but also their orientations. Reference is made to the description of Figs. 9-11 in which the positioning lines are referred to as cross lines 33 and 36.

Furthermore, by the graduation lines being printed in this way, the patient or caregiver can cut the hole closer to the centre of the template sheet 30. As a result, the opening of the base plate 12 which is cut based on this hole of the template sheet 30 can also be made better centred. Further, in the present embodiment, the template sheet 30 is printed with corner marks 30e of a square surrounding all of the contour lines 30a, 30b, and 30c. These corner marks 30e are used when cutting out part of the captured image data in the later explained step S13.

The patient or caregiver cuts the template sheet 30, for example, by cutting the template sheet 30 to obtain a hole of the same shape as the opening provided in the base plate of the previously used stoma device. This template sheet 30 may be cut by a person by hand by means of scissors or may be cut automatically using a mechanical device. The thus cut template sheet 30 for example has a hole 31 such as in Fig. 6.

On the other hand, when first using a stoma device or when the opening of the base plate of the previous used stoma device did not fit the stoma of the patient, the template sheet 30 is cut to match the shape of the actual stoma of the patient. At this time, since the template sheet 30 is cut with the hole 31, even if the stoma is raised up from the skin of the patient around the stoma, the template sheet 30 can be directly placed against the skin around the stoma to confirm that the shape of the hole 31 formed in the template sheet 30 matches the shape of the actual stoma, so fine adjustments can be made.

Next, as shown by step S12 of Fig. 4, the cut template sheet is sent to the cutting service. The template sheet 30 is preferably sent in a state where it is first disinfected after cutting of the template sheet 30, and then placed in a mailing envelope.

Next, at steps S13 to S16 in Fig. 4, a filled-in-part 32 of the same shape as the hole 31 formed in the template sheet 30 and of the shape matching the stoma of the patient is printed on a label. Specifically, the filled-in-part 32 is printed by the following steps S13 to S16.

First, at step S13, after the template sheet 30 in which the hole 31 is formed is sent to the cutting service, the template sheet 30 is run through a scanner so that image data of the template sheet 30 is loaded to a computer. At this time, it is preferable that the image data of the pattern 30 be loaded into the computer in a state enabling discrimination between the template sheet 30 and the hole 31 formed in the template sheet 30, for example, in a state where the template sheet 30 is colored while the hole 31 is not colored.

In particular, in the present embodiment, after the image data of the template sheet 30 as a whole is loaded into the computer, as shown in Fig. 7, part of this image data is cut out. Specifically, the square shape expressed by the corner marks 30e printed on the template sheet 30 explained above is cut out. Due to this, at the later explained step S16, printing of even unnecessary parts on the label is eliminated and the size of the image data can be reduced. The load on the database can therefore be reduced.

Next, at step S14, the part 31' of the image data of the template sheet 30, loaded by the scan at step S13, which corresponds to the hole 31 of the template sheet 30 is filled in. This part 31' may in the context of the present invention be referred to as a virtual hole 31'. In the present embodiment, as explained above, when loading the image data of the template sheet 30 in the computer, the data is loaded in a state enabling discrimination of the template sheet 30 and the hole 31 formed in the template sheet 30. Therefore, it is possible to easily and accurately select the virtual hole 31' (i.e. the part corresponding to the hole 31) of the template sheet 30. As a result, for example, as shown in Fig. 8, a black filled-in-part 32 is formed in the virtual hole 31' (i.e. the part of the image data corresponding to the hole 31 of the template sheet 30). Note that, in this specification, "filled in" or "filled-in-part" shall not only be understood as the insides of a given shape of a contour line are completely filled in but also as a given shape of a contour line and the inside given pattern.

Next, at step S15, the filled in image data, as shown in Fig. 9, is given cross lines 33. The centre of the cross lines 33 is positioned corresponding to the centre of gravity of the shapes expressed by the contour lines 30a, 30b, 30c, and 30d printed on the template sheet 30. In particular, in the present embodiment, the contour lines 30a, 30b, 30c, and 30d printed on the template sheet 30 are circular or elliptical and the centres of these shapes are the same point, so the centre of the cross lines 33 is made the same position as the points of the circles and ellipses.

At step S16, the image data prepared by steps S13 to S15, that is, the image data in which a filled-in-part 32 of the same shape as the hole 31 of the template sheet 30 and cross lines 33 are provided, is stored in a database. This image data is stored together with the customer number and other data together.

Next, at step S17, based on the image data which was stored in the database at step S16, the image of the image data is printed on the label 35. Therefore, the label 35 is printed with a filled-in-part 32 and cross lines 33 as shown in Figs. 9 and 10. The printing on the label 35 may be performed for each label or, for example, as shown in Fig. 10, may be performed for a plurality of connected labels such as three labels.

Further, in the present embodiment, when printing on the label 35, as the ink, a semi-resin ink is used. This ink has a higher environmental performance and has less of an effect on the human body compared with a wax-based ink etc. For this reason, even if part of the ink adheres to the base plate 12, it is possible to keep the effect on the environment or body to a minimum.

Next, at step S18, the label printed at step S17 is stuck to the unprocessed base plate 12. The string of labels such as shown in Fig. 10 is cut to separate one label at a time, then the label is stuck on an unprocessed base plate 12. At this time, the label 35 is stuck to the front surface of the base plate 12 (that is, on the protective sheet). Further, as shown in Fig. 11, the surface of the base plate 12 is printed with cross lines 36, while the label is stuck on the base plate so that the cross lines 33 printed on the label match with the cross lines 36 printed on the base plate 12. For this reason, it is possible to stick the label 35 accurately on the base plate 12 without any offset.

Note that, in the present embodiment, at step 15, the cross lines 33 are added to the image data, at step 17, the cross lines 33 are printed on the label 35, and, at step 18, the label 35 is stuck on the base plate 12 so that the cross lines 33 printed on the label 35 match with the cross lines 36 printed on the base plate 12. However, these lines do not have to be cross lines. They may be any shape of lines so long as enabling the label 35 and the base plate 12 to be positioned with each other. Therefore, if considering the inclusion of lines other than cross lines, the lines printed on the base plate 12 can be called "positioning reference lines" and the lines printed on the label 35 can be called "positioning lines".

Alternatively, as shown in Fig. 12, the surface of the base plate 12 may be marked to indicate sizes in the cross directions. If the surface of the base plate 12 is marked to indicate sizes in the cross directions in this way, it is possible to stick the label 35 on the base plate 12 so that the cross lines 33 printed on the label 35 match with the size indications.

After this, at step S19, the base plate 12 is cut along the contour of the filled-in-part 32 printed on the label stuck to the base plate 12. As explained above, the contour of the filled-in-part 32 printed on the label 35 becomes the same shape as the shape of the hole formed in the template sheet 30. Therefore, if the base plate 12 is cut along the contour of the filled-in-part 32 on the label 35, an opening of the same shape as the hole formed in the template sheet 30, that is, an opening of a shape matching the stoma of the patient, is formed in the base plate 12.

The base plate 12 formed with the opening in this way is sent from the cutting service to the patient.

Note that, in general, the shape of a stoma changes for about two months after surgery, but does not change much after the elapse of two months after surgery. Therefore, after the elapse of two months after surgery, the shape of the stoma remains the same. Along with this, the shape of the opening formed in the base plate 12 may also continue to remain as it is in some cases. In such a case, there is no need to repeat all of the above-mentioned steps 11 to 19. It is sufficient to repeat only steps S17 to S19.

In the present invention, it is possible to form in the base plate 12 an opening matching with a high precision the hole formed in the template sheet 30. As the hole in the template sheet is formed to fit the contours of the ostomy, the base plate will fit the ostomy.

As the method for measuring the precision of match of the hole formed in the template sheet 30 and the opening formed in the base plate 12, the following test method may be considered.

First, the base plate 12 and the template sheet 30 are centred with each other, then these are overlaid top and bottom to superpose the base plate 12 and the template sheet 30. This is done, for example, by aligning the graduation lines 30d printed on the template sheet 30 and the graduation lines printed on the base plate 12. In the state with the two superposed in this way, the error distance is determined as the distance between the opening of the base plate 12 and the hole of the template sheet 30. The error distance is the numerical value of the distance. First, the error distance is measured on the line extending radially in a 0° direction from the centres of the base plate 12 and the template sheet 30. Next, the error distance between the opening of the base plate 12 and the hole of the template sheet 30 on the line extending in a 45° direction from the centres is measured. Further, similar measurements are performed at 90°, 135°, 180°, 225°, 270°, and 315°. These 8 measurements are then averaged and this average is the accuracy of the cut relative to the hole.

In the alternative, starting from 0°, the distance between the cutting line of the base plate and the drawn line of the stoma template sheet is measured. This is repeated at 30°, 60°, 90°, 120°, 150°, 180°, 210°, 240°, 270°, 300°, and 330°. After this, the 12 values are averaged.

An alternative to the average of the measurements around the circle, is to calculate the sum of the determined numerical values of the error distances. It will be appreciated that in both cases the resulting number (i.e. the average or the sum) will be an indication of the precision of the cut of the base plate 12. Thus, the higher the number is, the lower is the precision of the cut and vice versa.

Note that, in the above embodiment explained using Fig. 4, the label 35 is stuck onto the base plate 12 and the base plate 12 to which the label 35 is stuck is cut (that is, Fig. 4, steps S18 and S19) by the cutting service. However, these tasks do not necessarily have to be performed by a cutting service. It is also possible for the cutting service to print the label 35 (step S17), then send the label 35 and the unprocessed base plate 12 to the patient. In this case, the label 35 is stuck onto the base plate 12 and the base plate 12 to which the label 35 is stuck is cut by someone other than the cutting service.

A "stoma" means an open hole in the digestive tract or the urinary tract created by ileostomy, urostomy, and colostomy. The adhesive material is typically an SIS or PIB type including a hydrophilic colloid. A "nurse" includes a home helper, health care provider, family, and the patient himself or herself.

At the time of use, a nurse draws the contour of the stoma of the patient on the stoma template sheet. Based on the stoma template sheet, the contour is electronically stored. When a new base plate is required, the contour is printed on the label, the label is attached to the base plate, and the label and base plate are cut along the contour.

In one embodiment, the base plate and the label are cut by scissors. This is a manual cut. This option shows the accuracy obtained by this cut model. That is, even with manual cutting of the base plate and label, a contour of a base plate exactly fitting the stoma of a user is provided.

In a preferable method, the stoma template sheet is modelled so as to match the base plate. By doing this, the nurse can match the stoma template sheet against the base plate attached to the patient. By the match of the stoma template sheet and the base plate, it is possible to make marks on the base plate and reflect the shape of the stoma hole of the stoma template sheet at a position relatively the same as on the base plate, for example, fitting of the base plate and the stoma pouch in the case of a two-piece system. As many patients have scars or irritation at the skin around the stoma, if the base plate is even slightly off in position, this will invite leakage or pain.

The accuracy is measured by the previously mentioned test method. The base plate is cut and is again matched with the stoma template sheet. Starting from 0°, the error distance between the cutting line of the base plate and the drawn line of the stoma template sheet is measured and its numerical value is determined. This is repeated at 45°,90°, 135°, 180°, 225°, 270°, and 315°. After this, the 8 numerical values are averaged. This value shows how inaccurate the cut is. It will be appreciated that the larger the average value is, the greater is the error between the cut at the base plate and the contour line of the stoma template sheet. When the value is small, the base plate and stoma template sheet are almost exactly superposed.

**Test results**

The abovementioned test has been carried out by the inventor. In the below, the field 'sum of numerical differences' corresponds to the sum of the numerical values of each of the differences. Accordingly for base plate 1 A, the sum is:

$$num(-2.373) + num(-3.300) + num(-2.074) + num(0.105) + num(2.520) + num(2.829) + num(1.928) + num(0.419) = 15.548$$

where 'num()' indicates that the numerical value (that is, the absolute value) of the number between the brackets is used in the calculation.

Table 1: Cutting guide 1 - prior art (cardboard measured)

| | 0° | 45° | 90° | 135° | 180° | 225° | 270° | 315° | Sum of numerical values | Average error distance |
|---|---|---|---|---|---|---|---|---|---|---|
| Cutting guide 1 | 10,151 | 12,1 | 11,63 | 8,958 | 5,926 | 5,447 | 5,407 | 7,178 | | |
| Base plate 1A | 7,778 | 8,8 | 9,556 | 9,063 | 8,446 | 8,276 | 7,335 | 7,597 | | |
| Difference between base plate and cutting guide | -2,373 | -3,3 | -2,074 | 0,105 | 2,52 | 2,829 | 1,928 | 0,419 | 15,548 | 1,944 |
| Base plate 1B | 8,741 | 9,323 | 9,333 | 8,802 | 7,704 | 7,386 | 7,037 | 7,858 | | |
| Difference between base plate and cutting guide | -1,41 | -2,777 | -2,297 | -0,156 | 1,778 | 1,939 | 1,63 | 0,68 | 12,667 | 1,583 |
| Base plate 1C | 8,667 | 9,271 | 9,333 | 8,696 | 8,075 | 7,647 | 7,186 | 7,858 | | |
| Difference between base plate and cutting guide | -1,484 | -2,829 | -2,297 | -0,262 | 2,149 | 2,2 | 1,779 | 0,68 | 13,680 | 1,710 |
| Base plate 1D | 8,741 | 9,323 | 10 | 9,063 | 8,149 | 7,911 | 6,889 | 7,7 | | |
| Difference between base plate and cutting guide | -1,41 | -2,777 | -1,63 | 0,105 | 2,223 | 2,464 | 1,482 | 0,522 | 12,613 | 1,577 |
| Base plate 1E | 8,889 | 9,062 | 9,111 | 8,907 | 8 | 7,752 | 7,556 | 8,014 | | |
| Difference between base plate and cutting guide | -1,262 | -3,038 | -2,519 | -0,051 | 2,074 | 2,305 | 2,149 | 0,836 | 14,234 | 1,779 |
| Average | | | | | | | | | 13,748 | 1,719 |

Table 2: Cutting guide 2 - prior art (cardboard measured)

| | 0° | 45° | 90° | 135° | 180° | 225° | 270° | 315° | Sum of numerical values | Average error distance |
|---|---|---|---|---|---|---|---|---|---|---|
| Cutting guide 2 | 10,225 | 13,881 | 15,778 | 11,682 | 9,185 | 7,282 | 7,111 | 8,014 | | |
| Base plate 2A | 10,445 | 12,1 | 13,037 | 10,164 | 9,037 | 9,376 | 9,111 | 9,116 | | |
| Difference between base plate and cutting guide | 0,22 | -1,781 | -2,741 | -1,518 | - 0,148 | 2,094 | 2 | 1,102 | 11,604 | 1,451 |
| Base plate 2B | 9,333 | 11,421 | 12,963 | 10,528 | 9,853 | 9,481 | 8,889 | 8,591 | | |
| Difference between base plate and cutting guide | -0,892 | -2,46 | -2,815 | -1,154 | 0,668 | 2,199 | 1,778 | 0,577 | 12,543 | 1,568 |
| Base plate 2C | 10,074 | 12,362 | 13,111 | 10,897 | 9,778 | 9,428 | 9,186 | 9,115 | | |
| Difference between base plate and cutting guide | -0,151 | -1,519 | -2,667 | -0,785 | 0,593 | 2,146 | 2,075 | 1,101 | 11,037 | 1,380 |
| Base plate 2D | 9,778 | 11,942 | 13,408 | 10,528 | 9,63 | 9,219 | 9,037 | 8,8 | | |
| Difference between base plate and cutting guide | -0,447 | -1,939 | -2,37 | -1,154 | 0,445 | 1,937 | 1,926 | 0,786 | 11,004 | 1,376 |
| Base plate 2E | 9,259 | 11,314 | 13,185 | 10,948 | 9,778 | 9,428 | 8,742 | 8,539 | | |
| Difference between base plate and cutting guide | -0,966 | -2,567 | -2,593 | -0,734 | 0,593 | 2,146 | 1,631 | 0,525 | 11,755 | 1,469 |
| Average | | | | | | | | | 11,589 | 1,449 |

Table 3: Cutting guide 3 - according to the invention

| | 0° | 45° | 90° | 135° | 180° | 225° | 270° | 315° | Sum of numerical values | Average error distance |
|---|---|---|---|---|---|---|---|---|---|---|
| Cutting guide 3 | 15,186 | 17,662 | 20,149 | 16,447 | 14,67 | 16,816 | 18,594 | 16,814 | | |
| Base plate 3A | 16,667 | 19,747 | 21,482 | 17,704 | 15,185 | 17,443 | 19,778 | 17,599 | | |
| Difference between base plate and cutting guide | 1,481 | 2,085 | 1,333 | 1,257 | 0,515 | 0,627 | 1,184 | 0,785 | 9,267 | 1,158 |
| Base plate 3B | 14,817 | 17,914 | 19,63 | 15,924 | 14,223 | 16,081 | 17,335 | 15,609 | | |
| Difference between base plate and cutting guide | -0,369 | 0,252 | -0,519 | -0,523 | -0,447 | -0,735 | -1,259 | -1,205 | 5,309 | 0,664 |
| Base plate 3C | 16,667 | 19,433 | 21,556 | 17,652 | 15,408 | 17,338 | 19,705 | 18,176 | | |
| Difference between base plate and cutting guide | 1,481 | 1,771 | 1,407 | 1,205 | 0,738 | 0,522 | 1,111 | 1,362 | 9,597 | 1,200 |
| Base plate 3D | 16,593 | 19,642 | 21,482 | 17,338 | 15,259 | 17,338 | 19,556 | 17,809 | | |
| Difference between base plate and cutting guide | 1,407 | 1,98 | 1,333 | 0,891 | 0,589 | 0,522 | 0,962 | 0,995 | 8,679 | 1,085 |
| Base plate 3E | 16,889 | 20,061 | 21,631 | 16,761 | 14,371 | 16,342 | 19,334 | 18,018 | | |
| Difference between base plate and cutting guide | 1,703 | 2,399 | 1,482 | 0,314 | -0,299 | -0,474 | 0,74 | 1,204 | 8,615 | 1,077 |
| Average | | | | | | | | | 8,293 | 1,037 |

Table 4: Cutting guide 4 - according to the invention

| | 0° | 45° | 90° | 135° | 180° | 225° | 270° | 315° | Sum of numerical values | Average error distance |
|---|---|---|---|---|---|---|---|---|---|---|
| Cutting guide 4 | 9,704 | 10,005 | 12,223 | 9,847 | 9,186 | 9,063 | 9,334 | 9,062 | | |
| Base plate 4A | 9,259 | 9,009 | 10,889 | 8,8 | 8,596 | 9,377 | 10,291 | 9,691 | | |
| Difference between base plate and cutting guide | -0,445 | -0,996 | -1,334 | -1,047 | -0,59 | 0,314 | 0,957 | 0,629 | 6,312 | 0,789 |
| Base plate 4B | 9,26 | 9,116 | 10,963 | 8,747 | 8,667 | 9,589 | 9,926 | 9,742 | | |
| Difference between base plate and cutting guide | -0,444 | -0,889 | -1,26 | -1,1 | - 0,519 | 0,526 | 0,592 | 0,68 | 6,010 | 0,751 |
| Base plate 4C | 9,112 | 8,958 | 11,482 | 9,481 | 8,963 | 9,742 | 10 | 9,271 | | |
| Difference between base plate and cutting guide | -0,592 | -1,047 | -0,741 | -0,366 | 0,223 | 0,679 | 0,666 | 0,209 | 4,523 | 0,565 |
| Base plate 4D | 9,408 | 8,695 | 10,815 | 8,433 | 8,15 | 9,64 | 10,296 | 9,376 | | |
| Difference between base plate and cutting guide | -0,296 | -1,31 | -1,408 | -1,414 | -1,036 | 0,577 | 0,962 | 0,314 | 7,317 | 0,915 |
| Base plate 4E | 9,111 | 8,8 | 10,741 | 8,957 | 8,889 | 9,901 | 9,927 | 8,852 | | |
| Difference between base plate and cutting guide | -0,593 | -1,205 | -1,482 | -0,89 | - 0,297 | 0,838 | 0,593 | -0,21 | 6,108 | 0,764 |
| Average | | | | | | | | | 6,054 | 0,757 |

Table 5: Cutting guide 5 - according to the invention

| | 0° | 45° | 90° | 135° | 180° | 225° | 270° | 315° | Sum of numerical values | Average error distance |
|---|---|---|---|---|---|---|---|---|---|---|
| Cutting guide 5 | 14,963 | 12,414 | 14,297 | 9,062 | 7,852 | 10,109 | 14,519 | 15,295 | | |
| Base plate 5A | 14,149 | 13,095 | 15,111 | 9,271 | 7,333 | 9,009 | 13,852 | 14,09 | | |
| Difference between base plate and cutting guide | -0,814 | 0,681 | 0,814 | 0,209 | -0,519 | -1,1 | -0,667 | -1,205 | 6,009 | 0,751 |
| Base plate 5B | 14,741 | 12,623 | 14,371 | 8,695 | 8,001 | 10,109 | 14,445 | 14,928 | | |
| Difference between base plate and cutting guide | -0,222 | 0,209 | 0,074 | -0,367 | 0,149 | 0 | -0,074 | -0,367 | 1,462 | 0,183 |
| Base plate 5C | 12,52 | 11,419 | 13,556 | 10,057 | 9,26 | 11,314 | 14,594 | 13,409 | | |
| Difference between base plate and cutting guide | -2,443 | -0,995 | -0,741 | 0,995 | 1,408 | 1,205 | 0,075 | -1,886 | 9,748 | 1,219 |
| Base plate 5D | 12,815 | 12,047 | 14,149 | 10,685 | 10,001 | 12,101 | 14,668 | 13,147 | | |
| Difference between base plate and cutting guide | -2,148 | -0,367 | -0,148 | 1,623 | 2,149 | 1,992 | 0,149 | -2,148 | 10,724 | 1,341 |
| Base plate 5E | 14,889 | 13,304 | 14,222 | 9,009 | 8,223 | 10,371 | 14,519 | 14,928 | | |
| Difference between base plate and cutting guide | -0,074 | 0,89 | -0,075 | -0,053 | 0,371 | 0,262 | 0 | -0,367 | 2,092 | 0,262 |
| Average | | | | | | | | | 6,007 | 0,751 |

The prior art measurements (tables 1 and 2) are cut after the stoma nurse faxes a drawing of the stoma shape to the cutting facility. The nurses use normal pens and lines are generally pretty good. When received, the shape is drawn on cardboard and scanned in to a data base. This is then printed and transferred to the cutting room with the correct shape to cut away from skin side.

The measurements of the cuts according to the invention (tables 3, 4 and 5) are cut after the stoma nurse cuts a hole in the template sheet. This sheet is send to the cutting facility as prescribed in figure 4.

These measurements confirms, that measuring with a template sheet according to the invention, the average error distance goes from 1.7 and 1.5mm to 1.1 mm and below. This is a marked improvement in the accuracy of the fit of the base plate to the area around the ostomy of the particular user.

## Claims

**1.** A method of forming an opening (14) in a base plate (12) of a stoma device (11), comprising the steps of:

- printing on a label (35) a filled-in-part (32) of the same shape as a hole of a shape formed on a template sheet (30) and matching the stoma of a patient;
- sticking the label (35) on which said filled-in-part (32) has been printed on to a base plate (12); and
- cutting the base plate (12) along the contour of the filled-in-part (32) of the label (35) stuck to said base plate (12).

**2.** A method of forming an opening (14) as set forth in claim 1, wherein said step of printing a filled-in-part (32) on the label (35) comprises the steps of:

- scanning a template sheet (30) on which a hole of a shape matching the stoma of the patient is formed;
- filling in the part corresponding to the hole in the image data of the scanned template sheet (30); and
- printing an image of the image data in which the part is filled in, on to a label (35).

**3.** A method of forming an opening (14) as set forth in claim 2, wherein the base plate (12) is printed with positioning reference lines (36),

- the method further comprises a step of adding positioning lines (33) corresponding to said positioning reference lines (36) to the image data,
- in the step of printing the image on the label (35), the positioning lines (33) are also printed on the label (35), and,
- in the step of sticking the label (35) on the base plate (12), the label (35) is stuck on the base plate (12) so that the positioning lines (33) of the label (35) are aligned with the positioning reference lines (36) of the base plate (12).

**4.** A method of forming an opening (14) as set forth in any one of claims 1 to 3, further provided with a step of cutting the template sheet (30) so as to form in the template sheet a hole (31) of a shape matching the stoma hole.

## Patentansprüche

**1.** Verfahren zum Bilden einer Öffnung (14) in einer Basisplatte (12) einer Stomavorrichtung (11), umfassend die folgenden Schritte:

- Drucken eines ausgefüllten Teils (32) mit derselben Form wie ein Loch mit einer Form, die auf einem Schablonenblatt (30) gebildet ist und zu dem Stoma eines Patienten passt, auf ein Etikett (35),
- Kleben des Etiketts (35), auf dem der ausgefüllte Teil (32) aufgedruckt ist, auf eine Basisplatte (12) und
- Schneiden der Basisplatte (12) entlang der Kontur des ausgefüllten Teils (32) des auf die Basisplatte (12) aufgeklebten Etiketts (35).

**2.** Verfahren zum Bilden einer Öffnung (14) nach Anspruch 1, wobei der Schritt des Druckens eines ausgefüllten Teils (32) auf das Etikett (35) folgende Schritte umfasst:

- Scannen eines Schablonenblatts (30), auf dem ein Loch mit einer Form gebildet ist, die zu dem Stoma des Patienten passt,
- Ausfüllen des Teils, der dem Loch entspricht, in den Bilddaten des gescannten Schablonenblatts (30) und

- Drucken eines Bilds der Bilddaten, in dem der Teil ausgefüllt ist, auf ein Etikett (35).

3. Verfahren zum Bilden einer Öffnung (14) nach Anspruch 2, wobei die Basisplatte (12) mit Positionierungsbezugslinien (36) bedruckt ist,

   - wobei das Verfahren ferner einen Schritt des Hinzufügens von Positionierungslinien (33), die den Positionierungsbezugslinien (36) entsprechen, zu den Bilddaten umfasst,
   - wobei die Positionierungslinien (33) im Schritt des Druckens des Bilds auf das Etikett (35) auch auf das Etikett (35) gedruckt werden, und
   - wobei das Etikett (35) im Schritt des Aufklebens des Etiketts (35) auf die Basisplatte (12) auf die Basisplatte (12) geklebt wird, so dass die Positionierungslinien (33) des Etiketts (35) auf die Positionierungsbezugslinien (36) der Basisplatte (12) ausgerichtet sind.

4. Verfahren zum Bilden einer Öffnung (14) nach einem der Ansprüche 1 bis 3, ferner umfassend einen Schritt des Schneidens des Schablonenblatts (30), um im Schablonenblatt ein Loch (31) mit einer Form zu bilden, die zu dem Stomaloch passt.

**Revendications**

1. Procédé de formation d'une ouverture (14) dans une plaque-support (12) d'un dispositif de stomie (11), comprenant les étapes suivantes :

   - imprimer, sur une étiquette (35), une zone ombrée (32) de la même forme qu'un trou dont la forme est définie sur une feuille formant gabarit (30) et est identique à la stomie d'un patient ;
   - coller l'étiquette (35), sur laquelle ladite zone ombrée (32) a été imprimée, sur une plaque-support (12) ; et
   - découper la plaque-support (12) le long du contour de la zone ombrée (32) de l'étiquette (35) collée sur ladite plaque-support (12).

2. Procédé de formation d'une ouverture (14) selon la revendication 1, dans lequel ladite étape d'impression d'une zone ombrée (32) sur l'étiquette (35) comprend les étapes suivantes :

   - numériser une feuille formant gabarit (30) sur laquelle est défini un trou dont la forme est identique à la stomie du patient ;
   - ombrer la zone correspondant au trou dans les données d'image de la feuille formant gabarit (30) numérisée ; et
   - imprimer, sur une étiquette (35), une image des données d'image dans lesquelles la zone est ombrée.

3. Procédé de formation d'une ouverture (14) selon la revendication 2, dans lequel des lignes de référence de positionnement (36) sont imprimées sur la plaque-support (12),

   - le procédé comprend en outre une étape consistant à ajouter aux données d'image des lignes de positionnement (33) correspondant auxdites lignes de référence de positionnement (36),
   - au cours de l'étape d'impression de l'image sur l'étiquette (35), les lignes de positionnement (33) sont également imprimées sur l'étiquette (35), et
   - au cours de l'étape de collage de l'étiquette (35) sur la plaque-support (12), l'étiquette (35) est collée sur la plaque-support (12) de telle sorte que les lignes de positionnement (33) de l'étiquette (35) soient alignées avec les lignes de référence de positionnement (36) de la plaque-support (12).

4. Procédé de formation d'une ouverture (14) selon l'une quelconque des revendications 1 à 3, comprenant en outre une étape consistant à découper la feuille formant gabarit (30) de façon à définir, dans la feuille formant gabarit, un trou (31) dont la forme est identique à l'orifice constituant la stomie.

FIG. 1

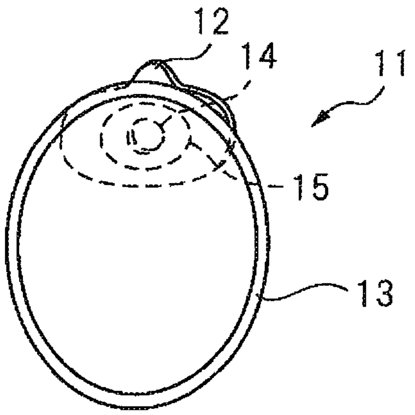


FIG. 2

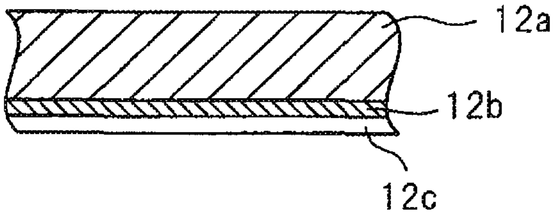


FIG. 3

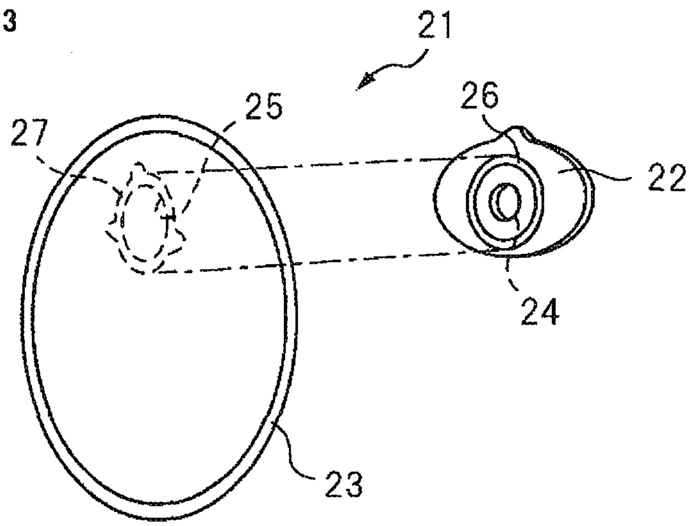

FIG. 4

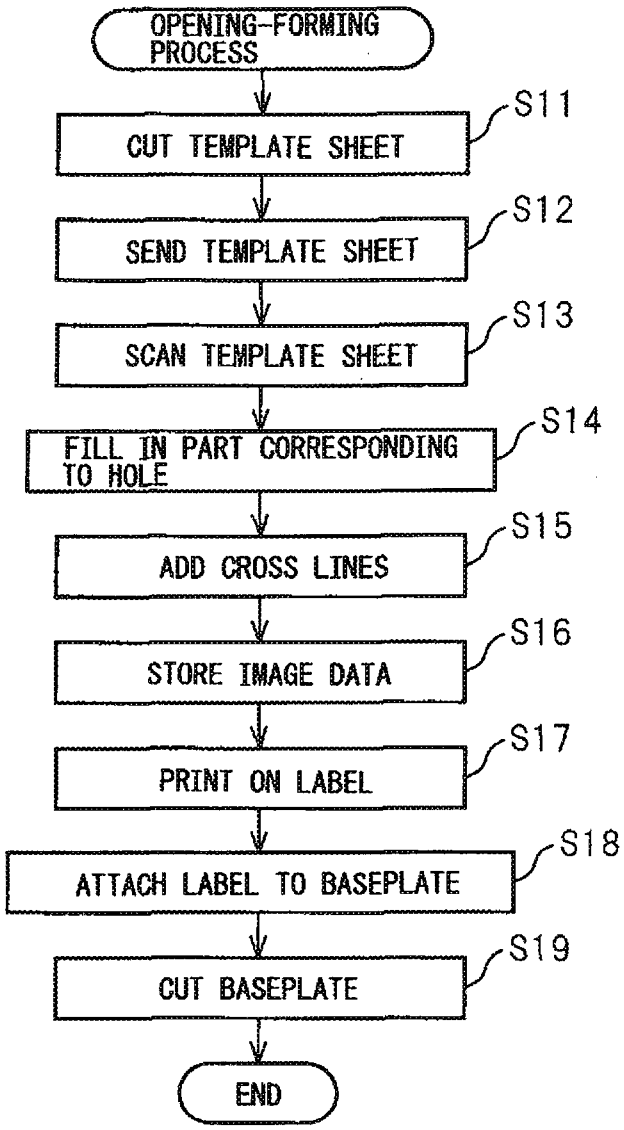
OPENING-FORMING PROCESS
CUT TEMPLATE SHEET
S11
SEND TEMPLATE SHEET
S12
SCAN TEMPLATE SHEET
S13
FILL IN PART CORRESPONDING TO HOLE
S14
ADD CROSS LINES
S15
STORE IMAGE DATA
S16
PRINT ON LABEL
S17
ATTACH LABEL TO BASEPLATE
S18
CUT BASEPLATE
S19
END

FIG. 5

PLAN VIEW

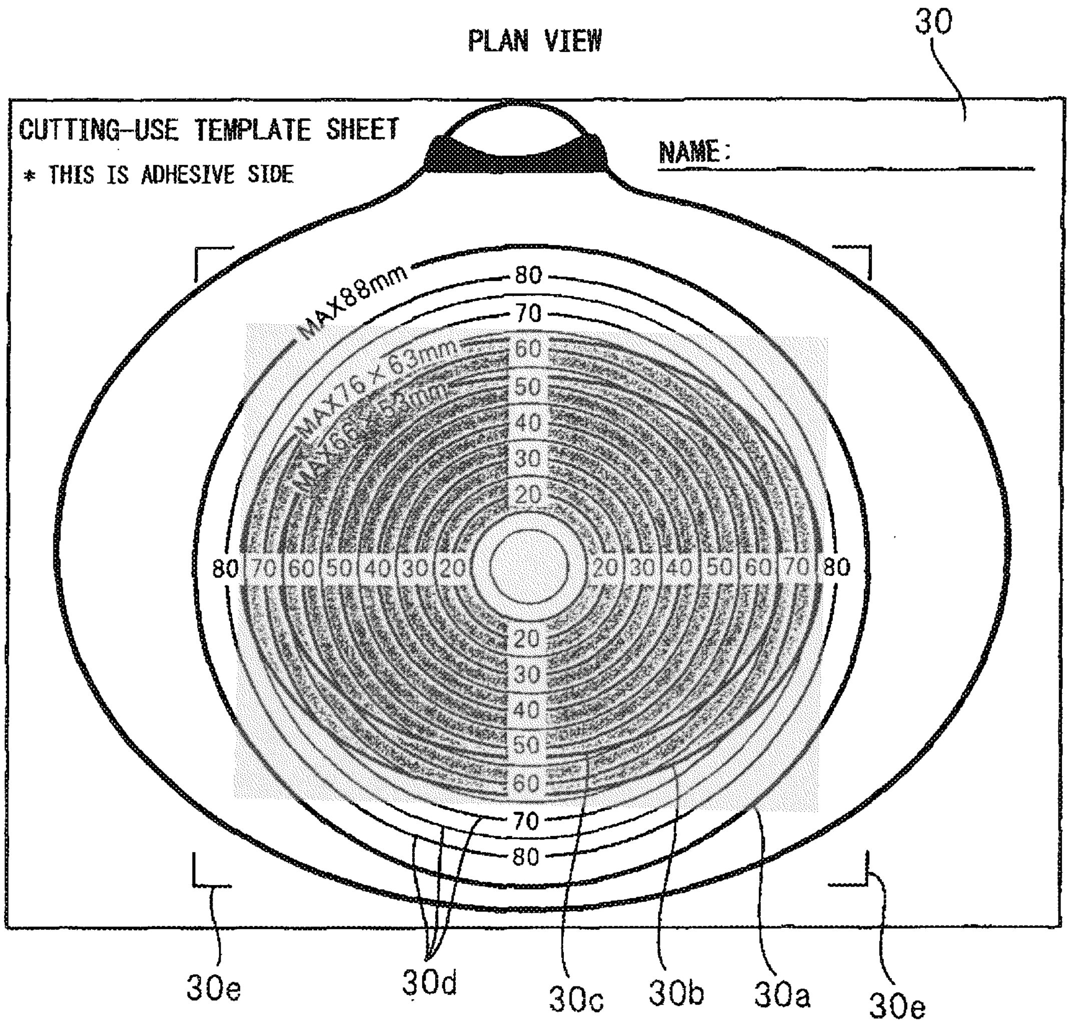


BOTTOM VIEW

FIG. 6

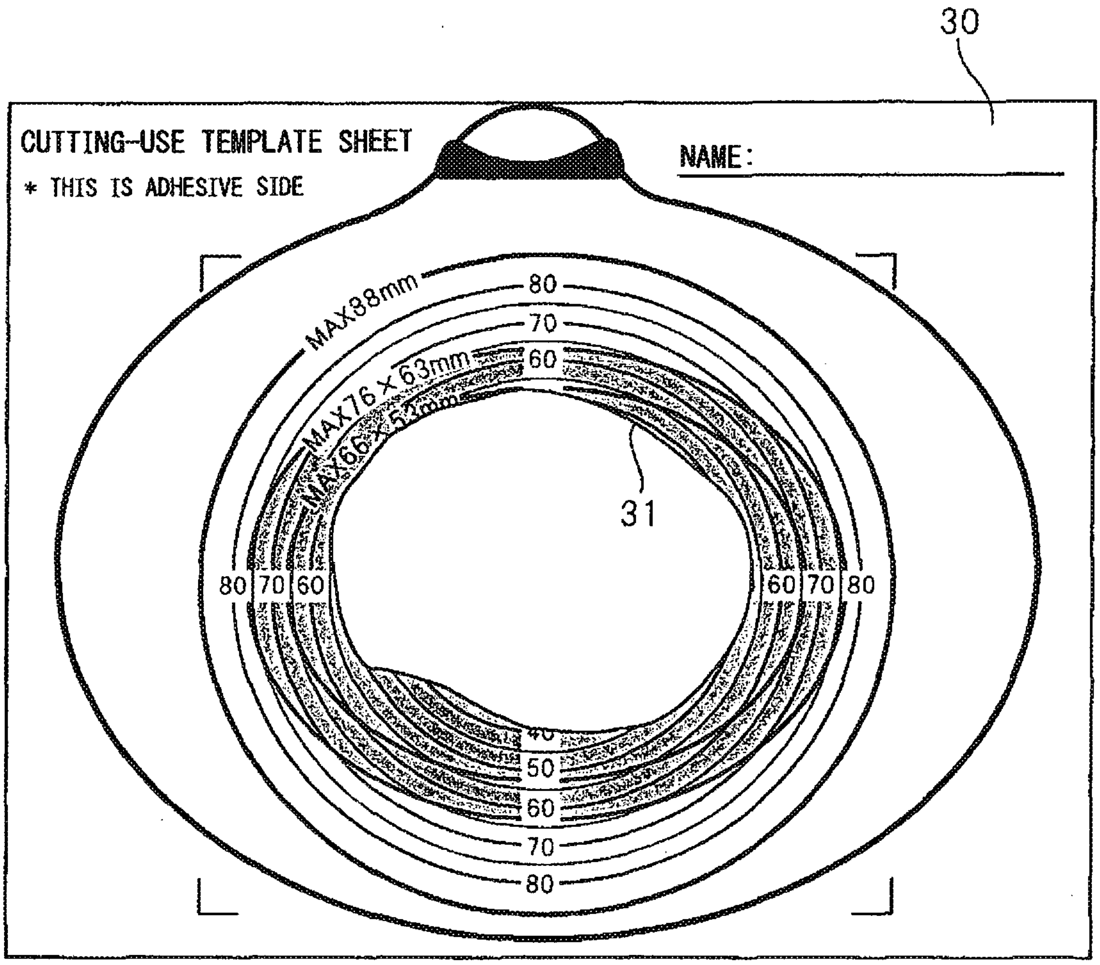
30
CUTTING-USE TEMPLATE SHEET
* THIS IS ADHESIVE SIDE
NAME:
MAX38mm
MAX76×63mm
80
70
60
50
40
31
80 70 60
60 70 80

FIG. 7

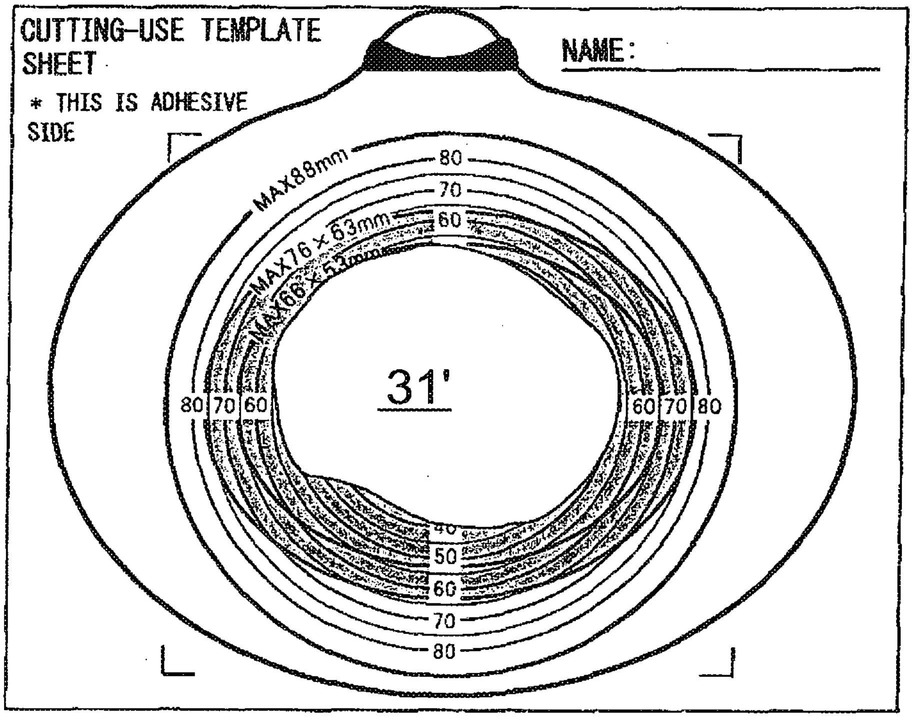
CUTTING-USE TEMPLATE SHEET
* THIS IS ADHESIVE SIDE
NAME:
MAX88mm
MAX76 × 63mm
MAX66 × 53mm
31'
80
70
60
50
40

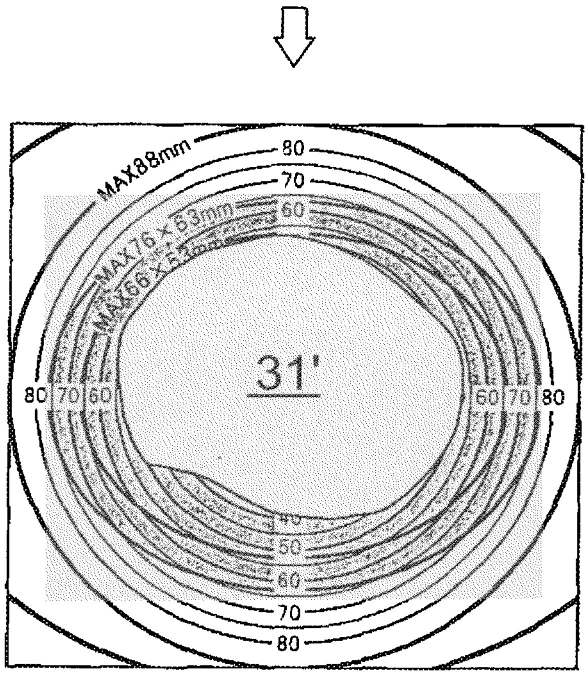
MAX88mm
MAX76 × 63mm
MAX66 × 53mm
31'
80
70
60
50
40

FIG. 8

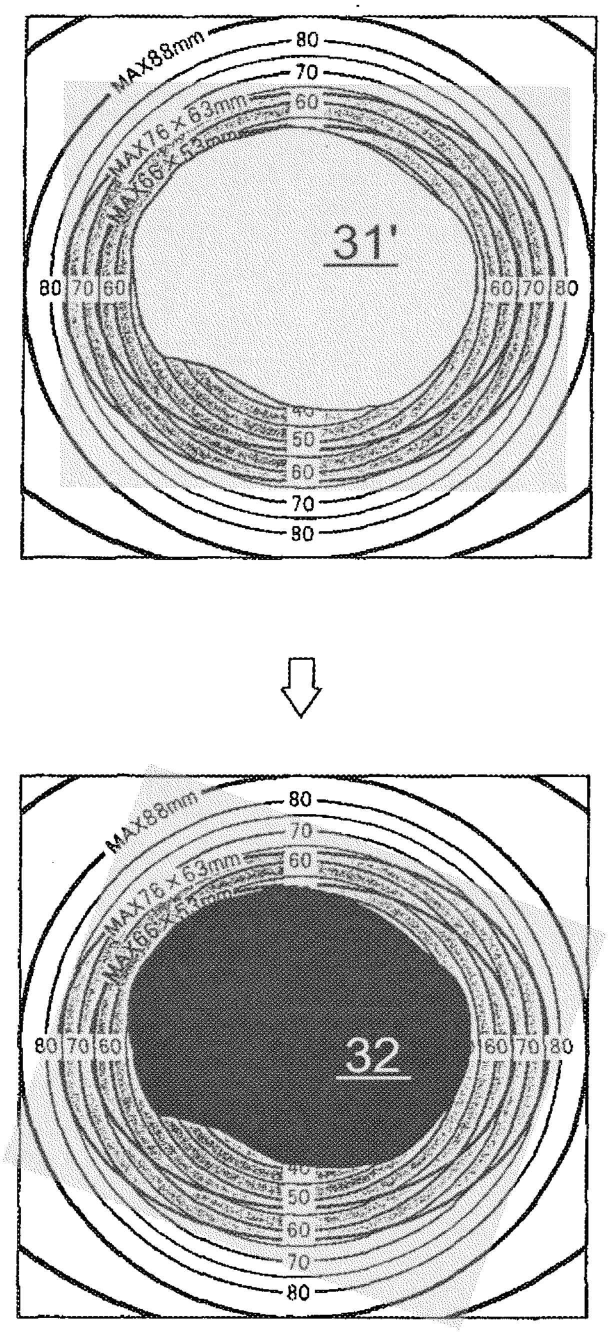
MAX88mm
80
70
60
MAX76 x 63mm
MAX66 x 53mm
31'
80 70 60
60 70 80
40
50
60
70
80
MAX88mm
80
70
60
MAX76 x 63mm
MAX66 x 53mm
32
80 70 60
60 70 80
40
50
60
70
80

FIG. 9

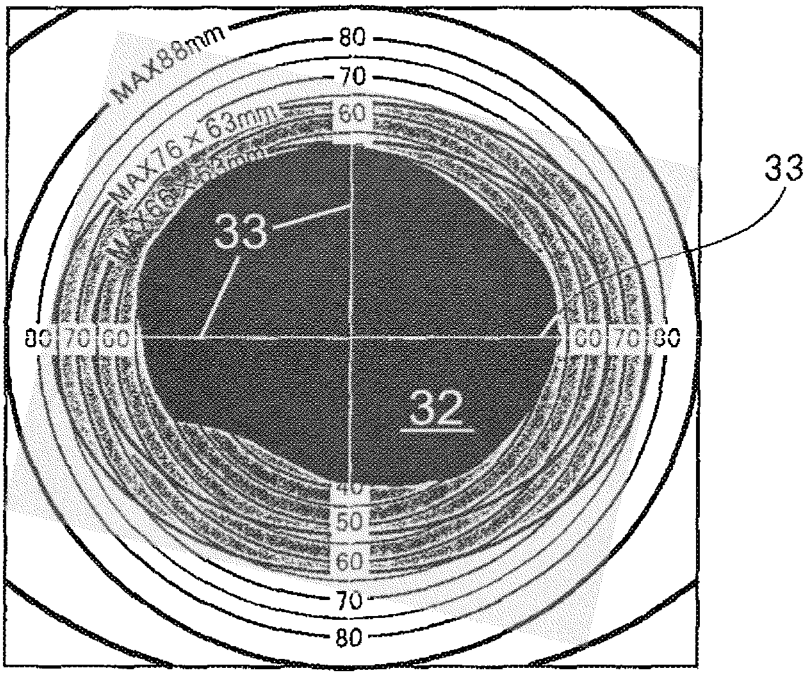

FIG. 10

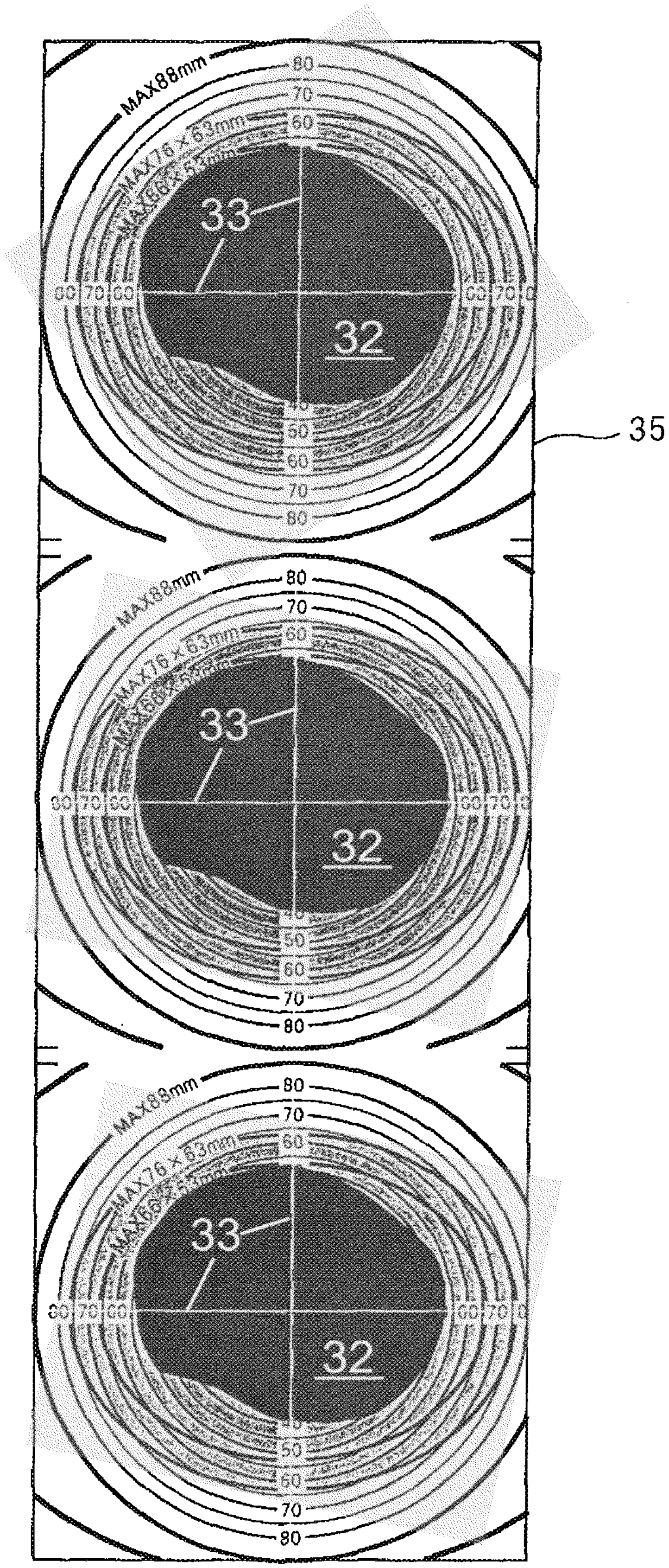

FIG. 11

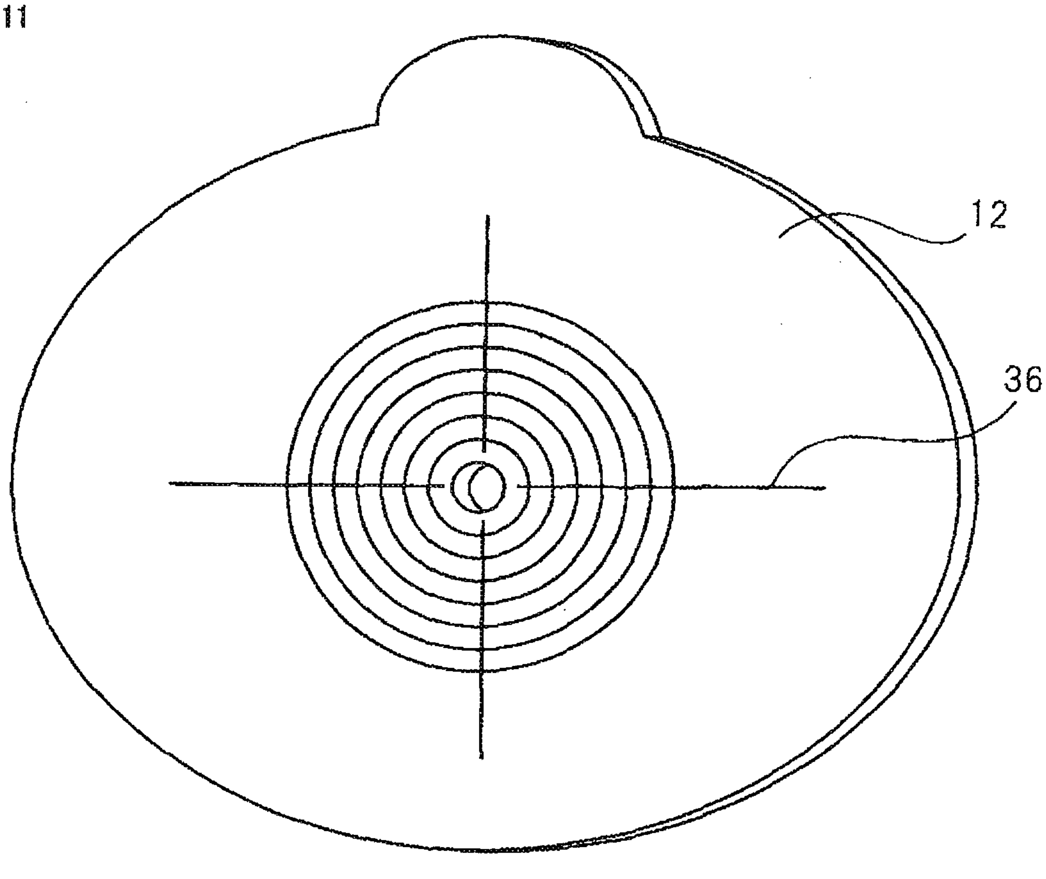

FIG. 12

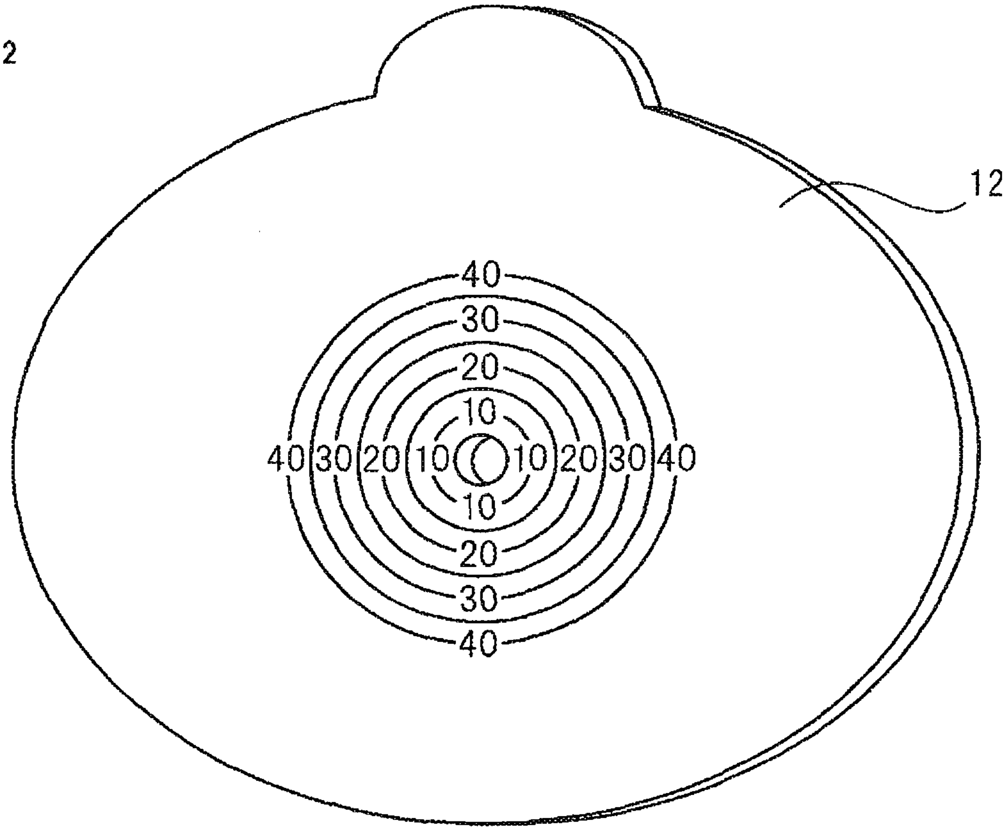

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 10028698 A
- WO 2006122565 A
- EP 0800804 A